# EUROPEAN PATENT APPLICATION

(11) **EP 3 919 092 A1**
(43) Date of publication of application: **08.12.2021**
(21) Application number: 20748624.2
(22) Date of filing: 21.01.2020
(51) Int. Cl.: A61L 27/50, C04B 38/00, B33Y 10/00, B33Y 70/00

(54) **SLURRY FOR PHOTOCURING 3D PRINTING, PREPARATION METHOD THEREFOR, AND METHOD OF USE THEREOF**

(30) Priority: 30.01.2019 US 201962798481 P
(71) Applicant: Kaohsiung Medical University, Kaohsiung City 80708 (TW)
(72) Inventor: WANG, Chih-Kuang, Kaohsiung, Taiwan 804 (TW); LEE, Chih-Yun, Township Chiayi, Taiwan 621 (TW); LEE, I-Chen, Kaohsiung, Taiwan 811 (TW); LIU, Che-Wei, Taipei, Taiwan 106 (TW); LIN, Che-Wei, Tainan, Taiwan 730 (CN)
(74) Representative: Lang, Christian
(86) International application number: PCT/CN2020/073381
(87) International publication number: WO 2020/156358

(57) **Abstract**

A method for preparing a slurry for photocuring 3D printing is provided, comprising the steps of: mixing monomer molecules of a thermosensitive hydrogel, a photocuring initiator, a crosslinking agent, a solvent, and a ceramic material to obtain the slurry. a method for manufacturing photocuring 3D printed articles is further provided, comprising using the slurry as a raw material, performing a 3D printing procedure by a photocuring 3D printer to obtain a green compact of a 3D printed article; and coating oil to the green compact of the 3D printed article, followed by heating and sintering the oil-coated article, to obtain the 3D printed article.

## Description

### FIELD OF THE INVENTION

The present invention relates to a method for preparing a slurry for photocuring 3D printing, which is characterized in that the slurry is obtained by mixing monomer molecules of thermosensitive hydrogel, a photocuring initiator, a crosslinking agent, a solvent, and a ceramic material.

### BACKGROUND OF THE INVENTION

As early as the 1980s, so-called "layered manufacturing" or "rapid prototyping" existed, which were used in mold opening, design, and calibration before industrial mass manufacturing. In recent years, the patents of these technologies have expired one after another, together with the progress of industrial development, this technology has gradually been widely used in daily life. The development of this technology makes it possible for unique redesign or customization requirements without cost constraints. Instead, it can be easily presented through this technology, which obviously overcomes the time and cost constraints, making the term "customization" become hot development trend.

The transformation of two-dimensional structure into three-dimensional presentation is one of the hot trends. Raw materials are one of the core technologies of 3D printing. At present, plastics, metals and ceramics are the three major mainstreams. However, in the next few years, it is inevitable to use multiple materials for printing. Compared with the early use of plastic, metal was used relatively late in the development of 3D printing. In the early days when plastic was the main raw material, 3D printing was not considered a machine manufacturing technology. But since the development of metal printing technology, this view has gradually changed. At present, there are two main methods for metal 3D printing: one method is to flatten the metal powder (or apply a thermal-sensitive plastic adhesive), and then directly use laser for selective sintering or bonding. In the latter case, the metal powder is bonded into a mold and then sintered in a hot furnace; another method is to extrude the molten metal through a printing nozzle, and then move the nozzle according to the design file to accurately shape the sprayed metal. In the past, consumer-grade 3D printers could only use plastic; however, with the advancement of printers and raw material technology, there are already some consumer-grade printers that can use raw materials of special materials, such as mixing metal powder with plastic. As for pure metal 3D printers, they cannot be classified as consumer 3D printers due to their high prices.

In addition to plastics and metals, ceramics and glass are also one of the raw materials for 3D printing, but they have application limitations. Regarding ceramic printing, there are currently two common methods: one method is to add a coagulant or photocuring resin to the ceramic powder and spray it through the print head; another method is to use a printing head to extrude the mud sticks to form a preform before sintering in the furnace. In these two methods, ceramic printing requires two or more than two stages of the process, namely printing, glaze coating and kiln sintering; this is usually accompanied by the following problems, such as easy deformation, preform drying required longer time and hard control for the coefficient of expansion.

Currently, one of the most important applications of ceramic printing is in the medical field. It is used to make parts of the human body such as artificial teeth or bones. The application of ceramic printing here is to scan the tooth model or bone shape with computer tomography first, and then print out 3D ceramic implants to reduce production costs and accelerate healing time, thereby achieving customized product production or reducing the operation time as the purposes. Although there are some porous phosphate bioceramic implants in the market, their mechanical properties are usually poor, and it is difficult to develop accurate and complex-shaped bone materials, resulting in high development costs, and it is impossible to further produce the bone material that conforms to and has a good continuous channel.

Previously, the inventors have developed a porous biphasic phosphate (HAp/TCP) bioceramic process using a new type of negative thermosensitive hydrogel pressure equalization and shrinkage technology to prepare bioceramics that not only have micropores but also have better mechanical stress. Through the control of the composition and sintering conditions, different proportions of biphasic phosphate (HAp/TCP) bioceramics could be obtained. At present, 3D bioprinting extrusion molding technology is still being developed commercially, and conventional dual-phase porous bone materials can be customized or standardized.

The current technologies, such as patents PCT/CN2016/108373, PCT/CN2017/096309, PCT/CN2018/082485, are based on a novel method of producing absorbable bioceramic scaffold materials by layered printing and applying them to the reconstruction of the skull and cement, which has biocompatibility, customized structure, and good mechanical properties. However, the characteristic of the current technology is that the ceramic slurry from the syringe is extruded for 3D printing and the green compact sample is molded with high temperature resistant oil. The hole phase in the dual continuous phase has a better grid-like hole structure only in the XY plane, but it is not easy to make a grid-like side hole structure on the vertical plane of the Z axis. The 3D printing molding method by using needle cylinder to extrude the ceramic slurry and to control the temperature rise generally still requires to go through a two-stage process, namely, the steps of forming and sintering.

At present, this kind of multi-layer 3D manufacturing method with extrusion process is still unable to produce small (for example, samples smaller than 0.8×0.8×0.8 cm3) and delicate hollow shapes; therefore, it is quite inconvenient to use in the production of small bone defects in orthopedics, tooth crowns and tooth roots.

### SUMMARY OF THE INVENTION

The present invention provides a method for preparing a slurry for photocuring 3D printing, which comprises the steps: mixing monomer molecules of a thermosensitive hydrogel, a photocuring initiator, a crosslinking agent, a solvent, and a ceramic material to obtain the slurry.

The present invention further provides a method for manufacturing photocuring 3D printed articles, which comprises the steps: (a) preparing a slurry, wherein the slurry is obtained by mixing monomer molecules of the thermosensitive hydrogel, a photocuring initiator, a crosslinking agent, a solvent, and a ceramic material; (b) using a photocuring 3D printer, which uses the slurry as a raw material to perform a 3D printing procedure to obtain a green compact of the 3D printed article; (c) coating oil to the green compact of the 3D printed article printed in step (b); and (d) Heating and sintering the green compact of the 3D printed article coated with the oil obtained in step (c) to obtain the 3D printed article.

### BRIEF DESCRIPTION OF THE DRAWINGS

**Figure 1** shows the finished products of 3D bioceramic components with various shapes, which are controlled by photocuring 3D printing technology to control the geometry of the 3D bioceramic components, such as hole size, spacing and pattern.
**Figure 2** shows the shrinkage of the bioceramic green compact after sintering with and without silicone oil.
**Figure 3** shows comparison of the shrinkage rate of the bioceramic green compacts coated with different oils and printed by photocuring 3D.
**Figure 4** shows the X-ray diffraction (X-Ray Diffraction; XRD) spectra of the bioceramics printed by 3D photocuring printer under different ratios of ceramic powder after sintering.
**Figure 5** shows the compressive strength of bioceramics printed by photocuring 3D printer.
**Figure 6** shows the cytotoxicity test of bioceramics printed by photocuring 3D printer.
**Figure 7** shows the results of the dispersibility of the bioceramic powder in the slurry.
**Figure 8** shows the bioceramic green compacts produced by pull-up photocuring 3D printing under different printing settings.
**Figure 9** shows a tooth model produced by photocuring 3D printing.
**Figure 10** shows the result of the drainage shrinkage test of the zirconia green compacts produced by photocuring 3D printing, wherein the green compacts are put into a solution mixed with silicone oil, double distilled water (DDW) and anhydrous alcohol (the above three solutions are all heated at 100°C in water for 1 hour), respectively.
**Figure 11** shows the test results of the viscosity of slurries with different solid content versus shear rate.

### DETAILED DESCRIPTION OF THE INVENTION

The feature of the present invention is based on the innovative negative thermosensitive paste ingredient formula used to build 3D ceramic components through digital light processing (DLP), that is, mixing micro and nano ceramic powders with negative thermosensitive monomer components (such as N-isopropylacrylamide (NiPAAm)), related crosslinking agents, co-crosslinking agents, photocuring initiators, solvents to form a slurry, which can be used in current commercial applications in photocuring 3D printers, to make 3D ceramic components.

The general preparation of 3D ceramic components is as follows: after the ceramic slurry being formed into a 3D ceramic green compact by photocuring 3D printing, then the green compact being sintered and densified at a high temperature to obtain the crude ceramic product. The final 3D ceramic component can be obtained by further processes such as mechanical surface processing.

In the current photocuring 3D printing technology, it is mainly divided into two types: stereolithography (SLA) and digital light processing (DLP). SLA is a laser drawing layer; and DLP is a projection drawing layer. Generally, the suspension (or slurry) used for SLA and DLP must meet the following requirements: (1) The suspension must have a certain fluidity to make up for the voids caused by layer-by-layer printing, so the viscosity of the suspension needs to be controlled by appropriate parameters (such as generally, the viscosity of the photocuring resin of the pull-up DLP is less than 2 to 8 mPa·s); (2) The optical layer printing must have sufficient curing depth under the operating conditions to push the object to be stacked as layer-by-layer formation; and (3) In order to have good sintering density, the more ceramic powder contained in the suspension, the better sintering density of ceramics. However, when ceramic powder is added to the organic photocuring resin, the particles will scatter the light, thereby affecting the depth of light-setting. In addition, adding ceramic powder will greatly increase the viscosity of the suspension. Therefore, factors such as curing depth and viscosity will make the 3D printed ceramic green compacts poor moldability.

As to printer parameter settings, the huge difference between the refractive index of the photocurable resin monomer and the ceramic particles results in a low penetration of ultraviolet (UV) beams. For example, under the same conditions and solid content, the solidification depth of the zirconia-containing suspension is four times less than that of the silica-containing suspension. In addition, the viscosity of the suspension and the cure depth (Cd) during the DLP curing process will also affect the printing results. When the viscosity is too high, cracks or defects will occur due to the strong separation between the newly solidified layer and the material tank, and if the viscosity is too low, it is not good for printing into a complete part; if the Cd is too large, the accuracy will be worse, if the Cd is too small, its mechanical properties will deteriorate. Further, the curing process is very sensitive to the exposure area and time. If it is not adjusted properly, the light scattering of the ceramic slurry will widen the final geometric shape and cause excessive growth of the green compact. Therefore, in the initial manufacturing process, a low energy dose is used as a curing strategy, and cracks occurred in the green compact can be reduced. Therefore, compared with silica and alumina, 3D printing of zirconia is more complicated and difficult. Furthermore, the parameters of the pull-up DLP molding machine and the sinking DLP molding machine are also very different.

Therefore, the present invention is to stir the negative thermosensitive hydrogel monomer molecules (NiPAAm) with the photocuring initiator, crosslinking agent and solvent until they are dissolved, and then stirred with the ceramic powder for 5 to 40 minutes to obtain the slurry for 3D printing, and the slurry is poured directly into the material tank for the photocuring 3D printer. The photocuring 3D printer uses UV digital projection bulbs. The printing slurry is cured after illumination, and is printed by surface forming, and the finished product is printed by stacking the plane and the plane; the fineness of the finished product partly depends on the pixel size of the planar light source. The printing process is as follow: digital-lightly projecting on a slurry mixed from negative thermosensitive hydrogel monomer molecules placed in the material tank and ceramic powder, adjusting lighting time of each layer and the moving speed of the printing stage and light-solid stacking and molding in a way of layer by layer to obtain the finished product.

The term "a" or "an" is used to describe the elements and components of the present invention. This term is only for convenience of description and to give the basic idea of the present invention. This description should be understood to include one or at least one, and unless clearly indicated otherwise, the singular also includes the plural. When used with the word "comprising" in the claims, the term "a" can mean one or more than one.

The term "or" means the same as "and/or".

The present invention provides a method for preparing a slurry for photocuring 3D printing, comprising the steps: mixing monomer molecules of a thermosensitive hydrogel, a photocuring initiator, a crosslinking agent, a solvent, and a ceramic material to obtain the slurry.

In a particular embodiment, the monomer molecules of the thermosensitive hydrogel, the photocuring initiator, the crosslinking agent, and the solvent are first stirred and mixed to obtain a mixed solution, and the mixed solution is then mixed with the ceramic material to obtain the slurry. Therefore, the monomer molecules of the thermosensitive hydrogel, the photocuring initiator, and the cross-linking agent will first dissolve in the solvent to form the mixed solution. Meanwhile, the mixed solution will not form a hydrogel, which is the state of a solution; then add the ceramic material to the mixed solution by uniform stirring to make the ceramic material high dispersion and uniformity in the mixture, and finally obtain a slurry with moderate viscosity and curability.

In another particular embodiment, the steps of the method for preparing the slurry of the present invention further comprises adding a dispersant. Therefore, the steps of the preparation method of the slurry for photocurable 3D printing technology of the present invention comprise: stirring and mixing the monomer molecules of the thermosensitive hydrogel, the photocuring initiator, the crosslinking agent, the dispersant, the solvent and the ceramic material to obtain the slurry. The main function of the dispersant is to promote the separation of particles in the suspension, avoid sedimentation or agglomeration, and enables the pre-dispersed particles to be stably dispersed in the medium. In a preferred embodiment, the dispersant comprises polyethylene glycol, poly(ethylene glycol) diacrylate (PEGDA), 2-hydroxyethyl methacrylate (HEMA), ammonium polyacrylate (APA), diammonium citrate (DAC), Dolapix CE 64, BYK w series (such as BYK w996 and BYK w969), Triton X series (such as Triton X-45 and Triton X-114), or combination thereof. Therefore, the monomer molecules of the thermosensitive hydrogel, the photocuring initiator, the crosslinking agent, and the dispersant are first dissolved in the solvent to obtain a mixed solution; then the ceramic material is added into the mixed solution, so that the ceramic material can be uniformly dispersed in the mixed solution to obtain a slurry with better properties.

In addition, a light absorber could further be added into the slurry of the present invention. The main function of the light absorber is to absorb and transfer light energy during the photocuring 3D printing process. In a particular embodiment, the steps of the preparation method of the slurry for photocuring 3D printing of the present invention further comprises adding a light absorber. In a preferred embodiment, the light absorber comprises 2-(2-hydroxy-5-methylphenyl) benzotriazole or Tinuvin P. Therefore, the preparation method of the slurry for photocuring 3D printing of the present invention can further add the dispersant and/or the light absorber.

In a particular embodiment, the ceramic material comprises hydroxyapatite, tricalcium phosphate, high-density alumina, zirconia, bioactive glass, carbide ceramic materials (such as silicon carbide), nitride ceramic materials (such as nitride Silicon), aluminum silicate, boride ceramic material, silicide ceramic material, or a combination thereof. In another particular embodiment, the ceramic material is in powder form. In a preferred embodiment, the ceramic material is ceramic powder. In a more preferred embodiment, the ceramic powder has a size of micrometer or nanometer level. Therefore, the slurry in the present invention can also be regarded as a ceramic slurry.

In a particular embodiment, the photocuring 3D printing includes stereolithography (SLA) 3D printing or digital light processing (DLP) 3D printing. In a preferred embodiment, the photocuring 3D printing is the digital light processing (DLP) 3D printing.

Since the application object of the slurry of the present invention is photocuring 3D printing technology or photocuring 3D printer, the solid content and viscosity of the slurry are very important. In a particular embodiment, the solid content of the slurry ranges from 10% to 60%. In a preferred embodiment, the solid content of the slurry ranges from 20% to 50%. In a more preferred embodiment, the solid content of the slurry ranges from 25% to 40%.

In another particular embodiment, the viscosity of the slurry ranges from 0.01 to 45 Pa·S. In a preferred embodiment, the viscosity of the slurry ranges from 0.5 to 40 Pa·S. In a more preferred embodiment, the viscosity of the slurry ranges from 1 to 30 mPa·S.

The present invention also provides a slurry comprising the monomer molecules of the thermosensitive hydrogel, a photocurable initiator, a crosslinking agent, a solvent and a ceramic material, wherein the slurry is prepared by mixing the monomer molecules of the thermosensitive hydrogel, the photocuring initiator, the crosslinking agent, the solvent and the ceramic material.

In a particular embodiment, the slurry further comprises a dispersant. In a particular embodiment, the dispersant comprises polyethylene glycol, poly(ethylene glycol) diacrylate (PEGDA), 2-hydroxyethyl methacrylate (HEMA), ammonium polyacrylate (APA), diammonium citrate (DAC), Dolapix CE 64, BYK w series (such as BYK w996 and BYK w969), Triton X series (such as Triton X-45 and Triton X-114), or a combination thereof.

In another particular embodiment, the slurry further comprises a light absorber. In a preferred embodiment, the light absorber comprises 2-(2-hydroxy-5-methylphenyl) benzotriazole or Tinuvin P.

In a particular embodiment, the solid content of the slurry ranges from 10% to 60%. In a preferred embodiment, the solid content of the slurry ranges from 20% to 50%. In a more preferred embodiment, the solid content of the slurry ranges from 25% to 40%.

In another particular embodiment, the viscosity of the slurry ranges from 0.01 to 45 Pa·S. In a preferred embodiment, the viscosity of the slurry ranges from 0.5 to 40 Pa·S. In a more preferred embodiment, the viscosity of the slurry ranges from 1 to 30 Pa·S.

The present invention further provides a method for manufacturing photocuring 3D printed articles, which comprises the steps: (a) preparing a slurry, wherein the slurry is obtained by mixing monomer molecules of the thermosensitive hydrogel, a photocuring initiator, a crosslinking agent, a solvent, and a ceramic material; (b) using a photocuring 3D printer, which uses the slurry as a raw material to perform a 3D printing procedure to obtain a green compact of the 3D printed article; (c) coating oil to the green compact of the 3D printed article printed in step (b); and (d) Heating and sintering the green compact of the 3D printed article coated with the oil obtained in step (c) to obtain the 3D printed article.

In a particular embodiment, the preparation method of the slurry in step (a) is further subdivided into stirring and mixing the monomer molecules of the thermosensitive hydrogel, the photocuring initiator, the crosslinking agent and the solvent to obtain a mixed solution, and mixing the mixed solution with the ceramic material to obtain the slurry.

As used herein, the term "thermosensitive hydrogel" refers to the polymer or monomer molecule of the present invention, which has a fluid-colloid temperature-induced reversible phase change; therefore, the polymer or the monomer molecule is in a specific temperature range, it will form a jelly or gel-like product, that is, a thermosensitive hydrogel. In a particular embodiment, the thermosensitive hydrogel is a negative thermosensitive hydrogel. In a preferred embodiment, the monomer molecules of the thermosensitive hydrogel are monomer molecules of the negative thermosensitive hydrogel. Generally, the temperature response range of the negative thermosensitive hydrogel is 30 to 50°C.

In another particular embodiment, the monomer molecules of the thermosensitive hydrogel comprise N-isopropylacrylamide (NiPAAm), N,N-Dimethylacrylamide, N-ethylmethacrylamide, methyl vinyl ether, 2-ethylhexyl vinyl ether, N-vinyl caprolactam, poly(organo phosphazene), N-isopropylacrylamide-methacrylic acid (NiPAAm-MAA), 1,6-hexanediol diacrylate (HDDA), 1,1,1-trimethylolpropane acrylate (TMPTA), isobornyl acrylate (IBOA), 2-hydroxyethyl acrylate (HEA), 2-hydroxyethyl methacrylate (HEMA), 2-phenoxyethyl acrylate (PHEA), isodecyl acrylate (IDA), Acrylic acid, difluorobenzophenone, Acrylamide, Tetra (ethylene glycol) diacrylate, or a combination thereof.

In another particular embodiment, the monomer molecules of the negative thermosensitive hydrogel comprise N-isopropylacrylamide, N,N-Dimethylacrylamide, N-ethylmethacrylamide, methyl vinyl ether, 2-ethylhexyl vinyl ether, N-vinyl caprolactam, poly(organo phosphazene), N-isopropylacrylamide-methacrylic acid, or a combination thereof.

The term "photocuring initiator" as used herein refers to a compound that undergoes a chemical change after receiving or absorbing external energy, and decomposes into free radicals or cations, thereby initiating a polymerization reaction. The absorption wavelength of the photocuring initiator in the present invention includes but is not limited to 350 nm to 420 nm. In a particular embodiment, the types of the photocuring initiator includes a free radical type photocuring initiator or a cationic photocuring initiator. The free radical type photocuring initiator includes but is not limited to acrylic acid or unsaturated polyester; the cationic photocuring initiator includes but is not limited to epoxy, oxetane or vinyl ether. In another particular embodiment, the photocuring initiator includes, but is not limited to, Lucirin TPO, IRGACURE 819, I2959, Darocur 1173, Irgacure 651, 2,2'-Azobis[2-(2-imidazolin-2-yl)propane]Dihydrochloride, Phenylbis(2,4,6-trimethyl-benzoyl)phosphine Oxide, UV6992, Cyracure UVI-6990, IC-250 or HCPK. In addition, the addition amount of the photocuring initiator is 0.5 to 5% by weight of the slurry.

The crosslinking agent herein is not particularly limited, as long as it can react with the functional groups on the monomer molecules of the thermosensitive hydrogel to initiate cross-linking. In a particular embodiment, the crosslinking agent includes polyurethane (PU), poly(ethylene glycol) diacrylate (PEGDA), polyurethane acrylate, polyurethane diacrylate (PU-diacrylate), tri-acrylate, methacrylic anhydride (MA), N,N'-Methylenebis(acrylamide), or a combination thereof.

The term "solvent" as used herein is a liquid that can dissolve solid, liquid, or gaseous solutes, followed by a solution. In a particular embodiment, the solvent includes a water solvent or an organic solvent. The term "organic solvent" as used herein refers to a type of solvent in which organic matter is the medium. Organic solvents can dissolve some water-insoluble substances, including many types of substances, such as alkanes, alkenes, alcohols, aldehydes, amines, esters, ethers, ketones, aromatic hydrocarbons, hydrogenated hydrocarbons, terpene olefins, halogenated hydrocarbons, heterocyclic compounds, nitrogen-containing compounds and sulfur-containing compounds, etc. In a particular embodiment, the organic solvent contains alcohols. In a preferred embodiment, the organic solvent includes methanol, ethanol, isopropanol, 1,5-pentanediol or benzyl alcohol. In a more preferred embodiment, the organic solvent is ethanol.

In a particular embodiment, the step (a) of the method for preparing the slurry further comprises adding a dispersant. Therefore, the slurry in the step (a) is prepared by mixing the monomer molecules of the thermosensitive hydrogel, the photocuring initiator, the crosslinking agent, the dispersant, the solvent, and the ceramic material. In another particular embodiment, the dispersant comprises polyethylene glycol, poly(ethylene glycol) diacrylate (PEGDA), 2-hydroxyethyl methacrylate (HEMA), ammonium polyacrylate (APA), Diammonium citrate (DAC), Dolapix CE 64, BYK w series (such as BYK w996 and BYK w969), Triton X series (such as Triton X-45 and Triton X-114), or a combination thereof.

In another particular embodiment, the step (a) of the method for preparing the slurry further comprises adding a light absorber. Therefore, the slurry in the step (a) is obtained by mixing the monomer molecules of the thermosensitive hydrogel, the photocuring initiator, the crosslinking agent, the light absorber, the solvent, and the ceramic material. In a particular embodiment, the light absorber comprises 2-(2-hydroxy-5-methylphenyl) benzotriazole or Tinuvin P. Therefore, the step (a) in the method for manufacturing a photocuring 3D printed article of the present invention could further add the dispersant and/or the light absorber to the slurry.

In another particular embodiment, the ceramic material includes hydroxyapatite, tricalcium phosphate, high-density alumina, zirconia, bioactive glass, carbide ceramic materials, nitride ceramic materials, aluminum silicate, boride ceramic material, silicide ceramic material, or a combination thereof. In another particular embodiment, the ceramic material is in powder form. In a preferred embodiment, the ceramic material is ceramic powder.

In a particular embodiment, the solid content of the slurry ranges from 10% to 60%. In a preferred embodiment, the solid content of the slurry ranges from 20% to 50%. In a more preferred embodiment, the solid content of the slurry ranges from 25% to 40%.

In another particular embodiment, the viscosity of the slurry ranges from 0.01 to 45 Pa·S. In a preferred embodiment, the viscosity of the slurry ranges from 0.5 to 40 Pa·S. In a preferred embodiment, the viscosity of the slurry ranges from 1 to 30 Pa·S.

As used herein, the terms "photocuring 3D printing", "photocuring 3D printing technology" or "photocuring 3D printer" refer to a technology or a machine in which the slurry is poured into the slurry tank and the printing platform is immersed in it, and then a UV laser or digital light projector is used to irradiate the beam on the printing platform. The irradiated slurry will be hardened and formed. The printing platform is raised from the slurry tank to be stacked layer by layer to obtain the finished product. Photocuring 3D printing includes two printing technologies: stereolithography (SLA) and digital light processing (DLP). The SLA technology uses two motors, called galvanometers (one on the X axis and one on the Y axis) to quickly aim the beam through the printing area, and the cured slurry is generated. This process is to slice the object into a large number of points and lines as the coordinate path of the laser reflected by the galvanometer. DLP uses a digital projection screen to flash a single image of each layer. The projected image is a digital image. Each layer of image is composed of square pixels, forming rectangular squares of each layer, called pixels; therefore, the fineness of the finished product will depend on the pixel size of the planar light source. In a particular embodiment, the photocuring 3D printer includes a stereolithography (SLA) photocuring 3D printer or a digital light processing (DLP) photocuring 3D printer. In a preferred embodiment, the photocuring 3D printer is the digital light processing (DLP) photocuring 3D printer. Therefore, in the 3D printing process performed by the photocuring 3D printer, the slurry is cured by ultraviolet radiation, and photo-cured slurry is further stacked and molded layer-by-layer to obtain the green compact of the 3D printed article.

According to the design file of the 3D printed article, the present invention can perform a 3D printing procedure through the photocuring 3D printer to print the green compact of the 3D printed article. In a particular embodiment, the green compact of the 3D printed article is an object with a geometric shape. Therefore, the printed green compact of the 3D printed article is further coated with oil, and then heated and sintered to obtain the final 3D printed article.

In a particular embodiment, the oil comprises polyglycol, silicone oil, fluorinated oil, phosphate ester, polyether, paraffin, dodecyl alcohol, olive oil, soybean oil, hydrocarbon mineral oil, liquid paraffin or synthetic hydrocarbon. In a preferred embodiment, the oil comprises silicone oil. In another particular embodiment, the oil coating method includes the oil fully or partially coating the green compact of the 3D printed article. In a preferred embodiment, the oil coating method includes the oil coating all the green compacts of the 3D printed article. Therefore, the dripped oil of the present invention can be higher or lower than the green compact of the 3D printed article.

The heating and sintering method of the present invention can be that the green compact of the 3D printed article coated with oil is put into a high temperature furnace for sintering. The sintering temperature is about 800°C to 1600°C, preferably 900°C to 1400°C, more preferably 1000°C to 1300°C. In a particular embodiment, the sintering time is 1 to 8 hours, preferably 3 to 6 hours. After the green compact of the 3D printed article is sintered, then the 3D printed article is obtained.

Since the slurry of the present invention is a ceramic slurry; therefore, the present invention is based on the ceramic slurry and is a finished product obtained by photocuring 3D printing, which is a 3D ceramic object or a 3D ceramic element. The main application of the present invention is in the field of biomedicine, such as making tooth models or bone-shaped implants; therefore, the 3D ceramic objects or 3D ceramic elements of the present invention can also be referred as 3D bioceramic objects or 3D bioceramic elements.

The ceramic slurry of the present invention is designed for photocuring 3D printers, so the viscosity of the ceramic slurry is lower, and the viscosity requirements of the sinking DLP and the pull-up DLP are also different. At the same time, the requirements for the uniformity of the dispersion of ceramic powder is higher. In the photocuring 3D printing process, the light source scans the slurry and simultaneously performs the photo-curing build-up of each layer. After the adjusted slurry is cured, the formed structure can be easily complicated with pore channels, and the surface is highly refined. Therefore, the 3D ceramic element finished product made from the slurry prepared by the present invention through light curing 3D printing has a finer fineness, an interpenetrating structure and side holes, a high degree of complexity in geometric shapes, and a smooth surface of the finished product, and with less stacking lines. In addition, the ceramic element designed by the present invention can be small item or large item. If the solid content of the slurry is higher than 40-55 % (vol%), the sintering shrinkage rate is less than 15%, such slurry is suitable for printing larger items (> ϕ5x10 cm).

### DESCRIPTION OF EMBODIMENTS

The following examples are non-limiting and only represent several aspects and characteristics of the present invention.

### Example 1

3-7g Polyurethane (crosslinking agent) and 1-3g photoinitiator (Phenylbis (2,4,6-trimethyl-benzoyl)phosphine Oxide, having UV absorption wavelength of 405mm were added into 70 ∼ 90g N-isopropylacrylamide (NiPAAm) monomer molecule of negative thermosensitive hydrogel, then 100 mL of absolute alcohol was added and stirred homogeneously; then mixed with hydroxyapatite (HAp) ceramic powder / β-tricalcium phosphate (β-TCP) ceramic powder, the ratio of the two ceramic powders was from 0:100 to 100:0, and obtained photo-curable printing slurry. The components of the slurry for photocuring 3D printing and the condition parameters of the photocuring 3D printer were shown in Table 1.

**Table 1. The components of the slurry for photocuring 3D printing and the condition parameters of the photocuring 3D printer**

| Solvent (mL) | monomer (NiPAAm) (g) | Cross-linking agent (g) | Photo-curing initiator (g) | Bottom layer number | Bottom layer settings | | | | General layer settings | | | |
|---|---|---|---|---|---|---|---|---|---|---|---|---|
| | | | | | Slice thickness (µm) | Curing time (s) | Draft height of each layer (mm) | Z axis moving speed (µm/s) | Slice thickness (µm) | Curing time (s) | Draft height of each layer (mm) | Z axis moving speed (µm/s) |
| 100 | 70∼90 | 3∼7 | 1∼3 | 6 | 50 | 60 | 6 | 1000 | 50 | 11 | 4 | 1000 |

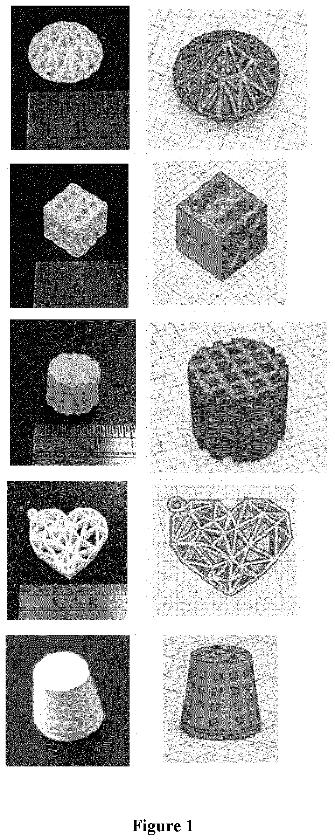

The 3D model image file was input into the photocuring 3D printer, and digital light processing (DLP) 3D printing is performed, the slurry is cured and formed into a bioceramic green compact through the setting of the ultraviolet (UV) module and the relevant irradiation time. The bioceramic green compact obtained after DLP printing was coated with silicone oil and then was sent into a high-temperature furnace for sintering at a temperature of 1150 to 1250°C to obtain the finished product of 3D bioceramic components. The 3D model image file and the finished product after sintering are shown in Figure 1.

The apparent density and apparent porosity of three groups of β-tricalcium phosphate bioceramics were tested. The apparent density was defined as the density measured with including closed pores in the object to be measured, and apparent porosity referred to the ratio of the volume of pores to the total volume of the material. The apparent density and the apparent porosity of the three groups of samples were measured by Archimedes method. The average apparent density of the three groups of β-tricalcium phosphate bioceramics (n=3) was 2.978g/cm³, and the average apparent porosity was 43.19% (Table 2), the apparent porosity could basically be adjusted through the design of the 3D model image file.

**Table 2. The apparent density and the apparent porosity of β-tricalcium phosphate bioceramics**

| | Sample 1 | Sample 2 | Sample 3 |
|---|---|---|---|
| Apparent density | 2.978g/cm³ | 2.980g/cm³ | 2.977g/cm³ |
| Apparent porosity | 43.98% | 40.72% | 44.87% |

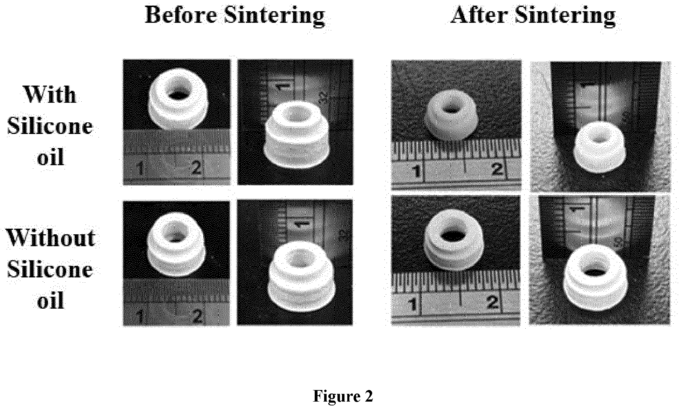

In addition, the difference in shrinkage rate of bioceramic green compacts coated with silicone oil and without silicone oil after sintering was compared. Figure 2 showed that after containing silicone oil, the sintering shrinkage rate of the bioceramics were significantly larger than without coated with silicone oil one. The result indicated that the densification degree of the bioceramics after sintering were also higher, and the mechanical property was better. Table 3 showed the shrinkage rates of the bioceramic green compacts coated with and without silicone oil after sintering.

**Table 3. Shrinkage rate of the bioceramic green compacts coated with and without silicone oil after sintering**

| | Before sintering | | Aftering sintering | | Shrinkage (%) | |
|---|---|---|---|---|---|---|
| | diameter | height | diameter | height | diameter | height |
| With silicone oil | 11.5 mm | 7 mm | 6 mm | 3 mm | 39% | 50% |
| Without silicone oil | 10 mm | 5 mm | 8 mm | 3 mm | 20% | 45% |

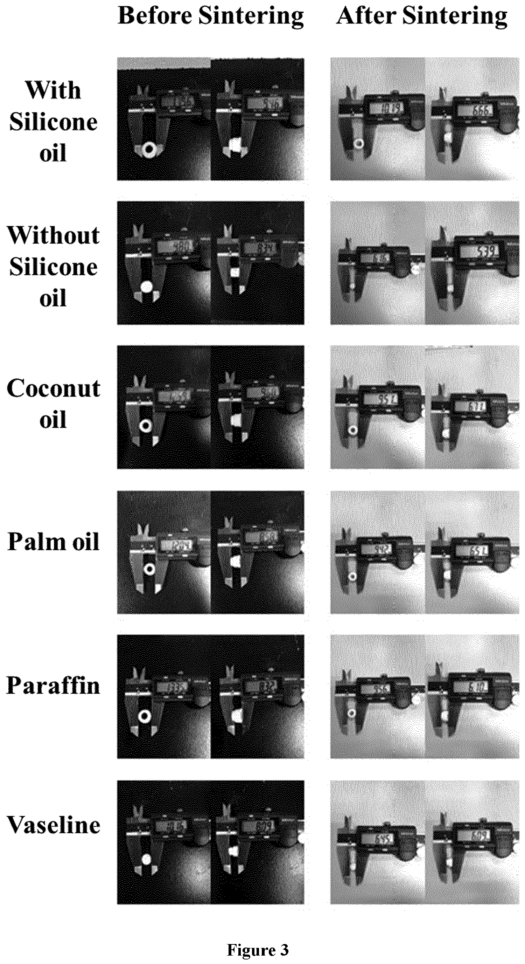

The shrinkage of the bioceramic green compact after sintering with different oil types were further compared (see Figure 3). It could be found that the diameter shrinkage of the oil-coated bioceramic green compact sample is greater than that of the non-oil-coated bioceramic green compacts sample, wherein the largest shrinkage in diameter and height was shown in the bioceramic green compacts sample coated with silicone oil. Table 4 showed the shrinkage rate of bioceramic green compacts coated with different oils after sintering.

**Table 4. Shrinkage rate of bioceramic green compacts coated with different oils after sintering**

| | Before sintering | | After sintering | | Shrinkage (%) | |
|---|---|---|---|---|---|---|
| | diameter | height | diameter | height | diameter | height |
| With Silicone oil | 11.96 mm | 9.46 mm | 10.19 mm | 6.66 mm | 15% | 30% |
| Without Silicone oil | 9.80 mm | 8.34 mm | 6.16 mm | 5.39 mm | 37% | 35% |
| Coconut oil | 12.59 mm | 9.60 mm | 9.51 mm | 6.71 mm | 24% | 30% |
| Palm oil | 12.04 mm | 8.58 mm | 9.42 mm | 6.51 mm | 22% | 24% |
| Paraffin | 13.35 mm | 8.32 mm | 9.56 mm | 6.10 mm | 28% | 27% |
| Vaseline | 10.16 mm | 8.09 mm | 6.45 mm | 6.09 mm | 37% | 25% |

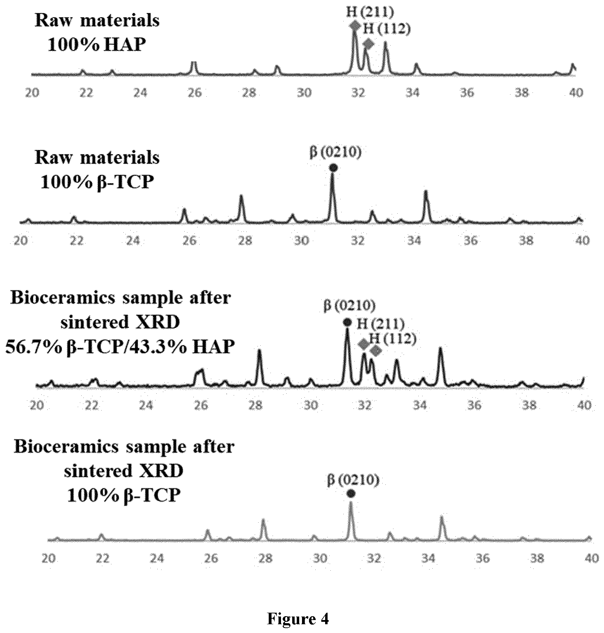

The phase structure of bioceramics were analyed by using X-ray diffraction. After the bioceramics are sintered at high temperature, the HAp in the composition was decomposed into TCP. Therefore, according to the addition of different ratios of β-TCP/HAp powder during the preparation of the printing material, the obtained final ratio of the two-phase ceramic was also different (Figure 4).

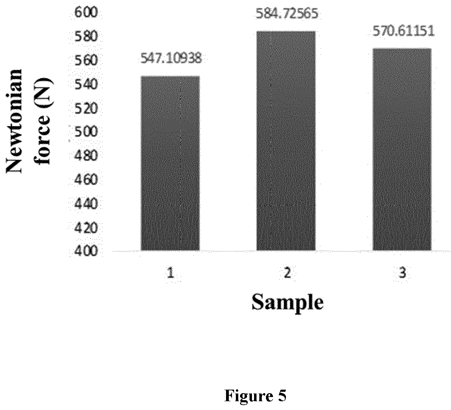

The compressive strength of three groups of bioceramics were tested. The sample size of three groups of bioceramics: average diameter was 8.30mm, average height was 5.65mm, average breaking strength was 567.48±19, Newtonian force (N) was 57.87kgf, average Young's modulus was 1870.36±613.05MPa (Figure 5), the compressive strength was calculated (σ=F/A=567.48N/13.5x10⁻⁶m²=42.03N/m²=42.03x10⁶Pa=42.03MPa, A = the average radius of the stent (2.07mm)²*3.1416=13.46mm²=1.35x10-5m²) was 42.03MPa. Table 5 showed the average values of the breaking strength and Young's modulus of the three groups of bioceramic samples.

**Table 5. The average value of the breaking strength and Young's modulus of the bioceramic samples**

| Sample | Breaking strength | Young's modulus |
|---|---|---|
| 1 | 547.11 | 1437.48 |
| 2 | 584.73 | 1601.72 |
| 3 | 570.61 | 2571.87 |
| Average value | 567.48 ± 19 | 1870.36 ± 613.05 |

The analysis of the chemical components of bioceramics was carried out by scanning electron microscope with Energy Dispersive Spectrometer (EDS) analysis technology to detect the elemental components of bioceramics, and the ratio of calcium to phosphorus (Ca/P) was measured to be 1.23 to 1.41, and the silicon (Si) content was less than 3.0. The heavy metal element analysis results of the bioceramics were shown in Table 6. The elements cesium (As), cadmium (Cd) and lead (Pb) did not exceed the standard specifications, and mercury (Hg) was not detected. Table 7 showed the chemical components analysis of bioceramics.

**Table 6. Analysis of heavy metal elements in bioceramics**

| Element | As | Cd | Hg | Pb |
|---|---|---|---|---|
| ppm | 0.061 | 0.009 | not detected | 0.0594 |

**Table 7. Chemical components analysis of bioceramics.**

| Element | Weight percentage (%) | Atom (%) |
|---|---|---|
| O K | 64.49 | 79.66 |
| Si K | 3.70 | 2.60 |
| P K | 14.19 | 9.05 |
| Ca K | 17.62 | 8.69 |
| Total | 100.00 | |

| | | |
|---|---|---|
| O: oxygen; K: potassium, Si: silicon; P: phosphorus; Ca: calcium | | |

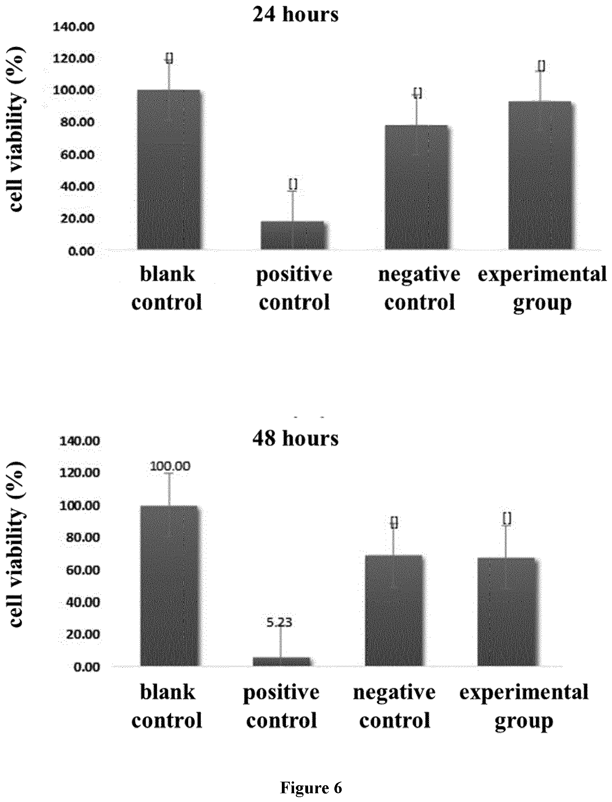

To further evaluate whether bioceramics had cytotoxicity to mouse fibroblasts (3T3 cell). Cytotoxicity analysis was measured by cell viability analysis (MTT assay) to test cell viability, that is, after 3T3 cell lines and material extracts were co-cultivated for 24 and 48 hours, the value of MTT was analyzed. The results of the experimental group were compared with tthose of the blank control group (blank control), positive control group (positive control), and negative control group (negative control). For the cell viability test analysis, the experimental results of the absorbance measured at 570 nm found that the cell viability of positive control group decreased by 76.6% compared with the cell viability of negative control group, and the cell viability of experimental group increased by 19.5% compared with the cell viability of negative control group (AL₂O₃). Further observation of the 48-hour MTT value showed that compared with the cell viability of negative control group, the cell viability of positive control group was reduced by 92.3%, and the cell viability of experimental group and the cell viability of negative control group were not significantly different. In summary, the MTT quantitative test results of the cell viability of experimental group did not reduce by more than 30% compared with the cell viability of negative control group., it confirmed that the bioceramics were not cytotoxic (Figure 6).

### Example 2

Because the density of zirconia=6g/cm³ and the refractive index=n20/D ∼ 2.2, it is a ceramic material that hard to be photocured to form a 3D shaped. Therefore, the present invention provided the monomer molecules of the negative thermosensitive hydrogel mixed with nano-grade Yttria-stabilized zirconia (YSZ) to develop a uniform, suitable viscosity and high-loaded zirconia suspension. The appropriate DLP 3D printing conditions (curing depth, single-layer exposure time) were solidified and molded, and the ceramic components could be drained and shrunk due to the material characteristics during the green compact sintering, and could avoid producing cracks and defects, so that the finished product could achieve compact and high mechanical strength.

### Production of photo-curing ceramic slurry

The materials used in the present invention were as follows:
3% yttria stabilized zirconia (3YSZ; Brand: HSY-3F-J): Put it in an oven at 60°C to dry before the experiment to prevent moisture from being adsorbed on the surface of zirconia;
Solvent: alcohol;
Dispersant: 2-hydroxyethyl methacrylate (HEMA), poly(ethylene glycol) diacrylate (PEGDA) or polyethylene glycol (PEG-400; PEG-600);
The monomer molecule of negative thermosensitive hydrogel: NiPAAm;
Crosslinking agent: polyurethane acrylate oligomer (PU2; Method: polyethylene glycol (PEG) + isophorone diisocyanate (IPDI)) +HEMA); and
Photocuring initiator: Phenylbis (2,4,6-trimethyl-benzoyl) phosphine Oxide.

After the monomer molecules of the negative thermosensitive hydrogel, alcohol, dispersant, crosslinking agent, and photocuring initiator (3% by weight of NiPAAm) were uniformly mixed in a proportion, the zirconia was added gradually. The mixture was put in an ultrasonic bath for 20 minutes and was further stirred by a homogenizer for 5 minutes to obtain a photo-curing ceramic slurry.

In the dispersibility experiment, the preparation method of the photo-curing ceramic slurry used in the present invention was as follows:

Alcohol: NiPAAm=10:9; and alcohol: PU2=10:0.64. After 10mL alcohol solution, 9g NiPAAm, 3% photocuring initiator and 0.64g PU2 were uniformly mixed, the HEMA with p-zirconia amount of 1%, 3%, 5%, and 10% were respectively added, stirred uniformly, added 1g 3YSZ, placed in an ultrasonic bath and shaked for 20 minutes, stirred with a homogenizer for 5 minutes (first stage 3 minutes, second stage 1.5 minutes,and third stage 0.5 minutes) and stood for observation.

The settings of DLP 3D printer were as follows:
The wavelength of 405 nm was used as the light source of the 3D printer, the single-layer curing time was 10 seconds, and the curing depth was set to 50µm and 58µm respectively for comparison. The 3D model was designed by Tinkercad to design a circle with a diameter of 16mm and a height of 0.5mm.

### Test results

First, the 10% HEMA dispersant, alcohol and zirconia powder (HSY-3F-J; DAIICHI KIGENSO KAGAKU KOGYO CO., LTD.) were mixed uniformly. The aggregation size of the particles was not only affected by the type of dispersant, but also changed due to the amount of dispersant. For example, when the amount of dispersant was low and not all particles adsorbed the dispersant on their surface, the aggregation caused due to the Van der Waals' force between each other. However, when the concentration of dispersion was too high, the particle size became larger due to the "micelle formation effect", and the viscosity of the suspension increased, which was not conducive to the photocuring process. Therefore, it was very important to find an appropriate ratio of the added dispersant.

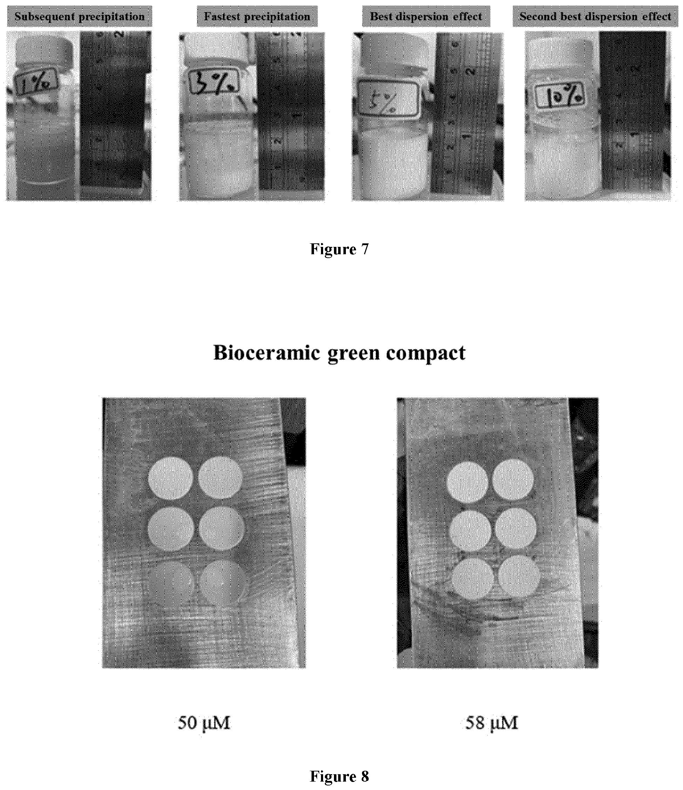

First, the alcohol and different proportions of HEMA dispersant were mixed, then zirconia was added. After 20 minutes of ultrasonic vibration, stirred with homogenizer for 5 minutes. After standing for 20 minutes, the best dispersion effect was observed at the 5% HEMA added, and the second better effect of dispersal was observed at the 10% HEMA added (Figure 7). Next, continuously stirred with a magnet, added NiPAAm and PU2 and mixed uniformly. Different results were found. The best dispersion effect was observed at the 10% HEMA dispersant added after standing for 30 minutes, followed by 5%, 1% and 3%. Therefore, the proportions of 10% and 5% were used to scale up the test.

30mL alcohol, 10% and 5% HEMA dispersant, 3g zirconia, 27g NiPAAm and 1.92mL PU2, 3% (0.81g) photoinitiator were mixed, and the precipitation was happened after 10g NiPAAm was added into 5% dispersant. However, it did not happen in 10g NiPAAm added with 10% dispersant. Therefore, photo-cured 3D printing was done with 10% dispersant.

The type of HEMA dispersant could greatly change the viscosity of the suspension. Follow-up tests of added 15%, 20%, 25% and 30% of the dispersant showed that the suspension effect of 20% dispersant added was better than that of 10% dispersant added, and the suspension effect of 25%, 30% dispersant added was equivalent to that of 20% dispersant added. However, the viscosity of the ceramic slurry was increased while increased the amount of HEMA. Therefore, while increasing the suspension load, it is necessary to maintain the appropriate viscosity of the ceramic slurry.

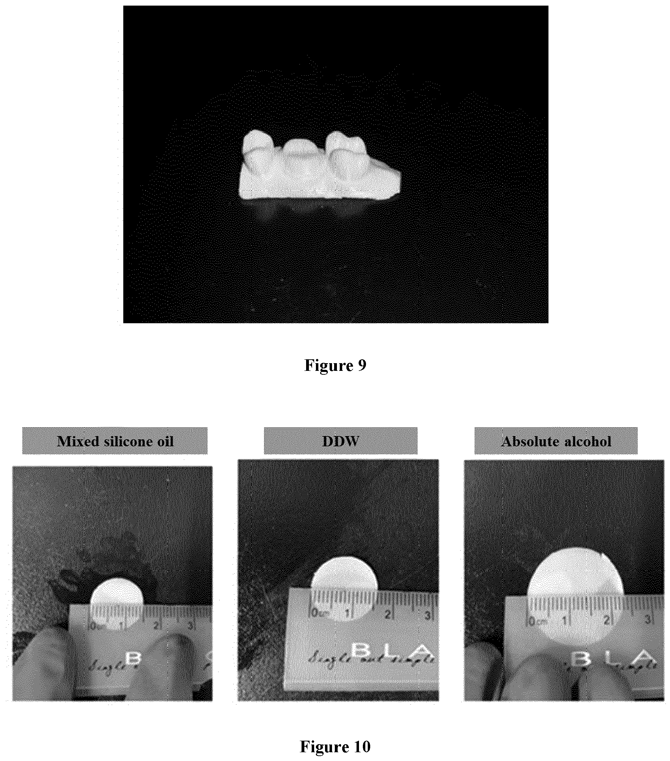

The present invention also tested the effect of the DLP 3D printer on the bioceramic green compacts at different curing depths. In the present invention, the single-layer curing time of the 3D printer was set to 10s, and the curing depth was set to 50 µm and 58 µm for comparison. As shown in Figure 8, there occurred irregular protrusions on the surface of the left side of the green compact, it might be caused by light scattering. If the printing thickness was increased, the shape could not be formed; the photocuring on the right was better. Figure 9 showed the results of the tooth model printed with this parameter.

The drainage shrinkage test was further performed in the present invention. The drainage shrinkage test of the present invention indicated that a 16mm diameter zirconia green compact was placed into a mixture of silicone oil, double distilled water (DDW) and absolute alcohol, respectively, heated to 100°C, and took it out for observation after 1 hour. It could be observed that the diameter of the green compact immersed in mixed silicone oil was reduced to 15mm; the diameter of the green compact immersed in double distilled water and heated still maintained 16mm; the diameter of the green compact immersed in absolute alcohol increased to 23mm. According to the above experimental results, it could be seen that the soaking and mixing of silicone oil contributed to the drainage shrinkage of the negative thermosensitive hydrogel (Figure 10). The present invention proved that adjusted the ratio of the photocuring ceramic slurry to suitable DLP photocuring printing conditions, and the ceramic slurry had the possibility of forming a 3D structure. The drainage shrinkage test was performed on ceramic green compacts with low solid content, which proved that adding NiPAAm and soaking the mixed silicone oil could still exhibit drainage shrinkage. Therefore, the slurry of the present invention could be applied to a sinking photocuring 3D printer, that is, a high solid content zirconia slurry (>30-55 vol%) could also be applied to a photocuring 3D printer for multilayer printing of ceramic components.

### Example 3

The present invention used 100% hydroxyapatite (HAp) and 100% β-tricalcium phosphate (β-TCP) slurries with different solid content to perform 3D printing sintering tests. The results of the sintering test showed that if the solid content of the slurry was too low, the printed objects were prone to generate voids during the sintering process; on the contrary, too high solid content made the material too heavy and difficult to shape during the photocuring 3D printing process. The printable and sintered yields of the finished products printed with different solid contents of hydroxyapatite and β-tricalcium phosphate slurries were shown in Table 8 and Table 9. Therefore, the solid content of 100% hydroxyapatite slurry for photocuring 3D printing was preferably 20 to 27%, and the solid content of 100% β-tricalcium phosphate slurry for photocurable 3D printing was 32.8 to 39.4%.

**Table 8. Printable and sintered yield test of hydroxyapatite slurry with different solid content**

| Solid content | 10.0% | 20.0% | 24.0% | 27.0% | 30.0% |
|---|---|---|---|---|---|
| Product yield (printing) | 20.5% | 65% | 82.6% | 75.2% | 10% |
| Product yield (sintering) | 5% | 43.2% | 75.2% | 70.3% | 0% |

**Table 9. Printable and sintered yield test of different solid content of β-tricalcium phosphate slurry**

| Solid content | 24.6% | 32.8% | 35.0% | 39.4% | 41% |
|---|---|---|---|---|---|
| Product yield (printing) | 33% | 83.2% | 91.7% | 62.2% | 2% |
| Product yield (sintering) | 12% | 65% | 76.3% | 46.8% | 0% |

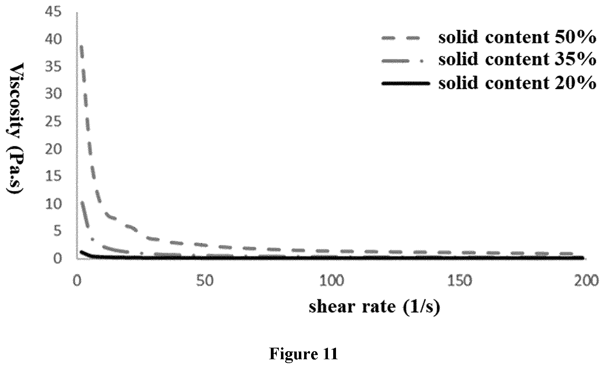

The material properties required for photocuring showed shear thinning characteristics. The viscosity of the slurry with a solid content of 50%, 35%, and 20% were tested with a rheometer. In the shear rate range of 1 to 200s⁻¹, the maximum viscosity of the slurry with a solid content of 50%, 35%, and 20% were 38.6 Pa·s, 10.25 Pa·s and 1.12 Pa·s, respectively (Figure 11). The viscosity of the slurry used for printing was decreased with the increasing of shear rate or shear stress, which proved that it had the characteristics of shear thinning; it was because the entanglement of polymer with each other at low flow rate, and the viscosity became larger, when the flow rate was increased, the shear stress disrupted the polymer chain structure, reduced the winding phenomenon and reduced the viscosity; and according to the experiment, it could be found that when the solid content was increased, although the viscosity increased, the shear thinning characteristics were still exhibited, so it was proved that increasing the solid content did not affect the curing ability. In this embodiment, the viscosity of the slurry suitable for photocuring 3D printing was in the range of 1.12 to 38.6 Pa s.

The above-mentioned embodiments are merely illustrative to illustrate the effects of the present invention, and to illustrate the technical features of the present invention, rather than to limit the protection scope of the present invention. Any person skilled in the art can easily make changes or arrangements without departing from the technical principle and spirit of the present invention, and they fall within the scope of protection claimed by the present invention. Therefore, the protection scope of the present invention is listed in the appended claims.

## Claims

1. A method for preparing a slurry for photocuring 3D printing, which comprises the steps: mixing monomer molecules of a thermosensitive hydrogel, a photocuring initiator, a crosslinking agent, a solvent, and a ceramic material to obtain the slurry.

2. The method of claim 1, wherein the monomer molecules of the thermosensitive hydrogel comprise: N-isopropylacrylamide (NiPAAm), N,N-Dimethylacrylamide, N-ethylmethacrylamide, methyl vinyl ether, 2-ethylhexyl vinyl ether, N-vinyl caprolactam, poly(organo phosphazene), N-isopropylacrylamide-methacrylic acid (NiPAAm-MAA), 1,6-hexanediol diacrylate (HDDA), 1,1,1-trimethylolpropane acrylate (TMPTA), isobornyl acrylate (IBOA), 2-hydroxyethyl acrylate (HEA), 2-hydroxyethyl methacrylate (HEMA), 2-phenoxyethyl acrylate (PHEA), isodecyl acrylate (IDA), Acrylic acid, difluorobenzophenone, Acrylamide, Tetra (ethylene glycol) diacrylate, or a combination thereof.

3. The method of claim 1, wherein the solvent comprises a water solvent or an organic solvent.

4. The method of claim 1, wherein the ceramic material comprises hydroxyapatite, tricalcium phosphate, high-density alumina, zirconia, bioactive glass, carbide ceramic materials, nitride ceramic materials, aluminum silicate, boride ceramic material, silicide ceramic material, or a combination thereof.

5. The method of claim 1, further comprising adding a dispersant.

6. The method of claim 1, further comprising adding a light absorber.

7. The method of claim 1, wherein the solid content of the slurry ranges from 10% to 60%.

8. The method according to claim 1, wherein the viscosity of the slurry ranges from 0.01 to 45 Pa·S.

9. A slurry prepared by the method of claim 1.

10. A method for manufacturing photocuring 3D printed articles, which comprises the steps: (a) preparing a slurry, wherein the slurry is obtained by mixing monomer molecules of the thermosensitive hydrogel, a photocuring initiator, a crosslinking agent, a solvent, and a ceramic material; (b) using a photocuring 3D printer, which uses the slurry as a raw material to perform a 3D printing procedure to obtain a green compact of the 3D printed article; (c) coating oil to the green compact of the 3D printed article printed in step (b); and (d) Heating and sintering the green compact of the 3D printed article coated with the oil obtained in step (c) to obtain the 3D printed article.

11. The method of claim 10, wherein the monomer molecules of the thermosensitive hydrogel comprise: N-isopropylacrylamide (NiPAAm), N,N-Dimethylacrylamide, N-ethylmethacrylamide, methyl vinyl ether, 2-ethylhexyl vinyl ether, N-vinyl caprolactam, poly(organo phosphazene), N-isopropylacrylamide-methacrylic acid (NiPAAm-MAA), 1,6-hexanediol diacrylate (HDDA), 1,1,1-trimethylolpropane acrylate (TMPTA), isobornyl acrylate (IBOA), 2-hydroxyethyl acrylate (HEA), 2-hydroxyethyl methacrylate (HEMA), 2-phenoxyethyl acrylate (PHEA), isodecyl acrylate (IDA), Acrylic acid, difluorobenzophenone, Acrylamide, Tetra (ethylene glycol) diacrylate, or a combination thereof.

12. The method of claim 10, wherein the solvent comprises a water solvent or an organic solvent.

13. The method of claim 10, wherein the ceramic material comprises hydroxyapatite, tricalcium phosphate, high-density alumina, zirconia, bioactive glass, carbide ceramic materials, nitride ceramic materials, aluminum silicate, boride ceramic material, silicide ceramic material, or a combination thereof.

14. The method of claim 10, wherein the oil comprises polyglycol, silicone oil, fluorinated oil, phosphate ester, polyether, paraffin, dodecyl alcohol, olive oil, soybean oil, hydrocarbon mineral oil, liquid paraffin or synthetic hydrocarbon.

15. The method of claim 10, wherein the solid content of the slurry ranges from 10% to 60%.

16. The method of claim 10, wherein the viscosity of the slurry ranges from 0.01 to 45 Pa·S.
